# EUROPÄISCHE PATENTSCHRIFT

(11) **EP 0 565 717 B1**
(45) Veröffentlichungstag und Bekanntmachung des Hinweises auf die Patenterteilung: **03.07.1996**
(21) Anmeldenummer: 91919157.7
(22) Anmeldetag: 06.11.1991
(51) Int. Cl.: A61F 13/46, B29C 51/00

(54) **FLEXIBLE, LUFTDURCHLÄSSIGE KUNSTSTOFFOLIE**
FLEXIBLE AIR-PERMEABLE PLASTIC SHEET
FEUILLE PLASTIQUE SOUPLE PERMEABLE A L'AIR

(43) Veröffentlichungstag der Anmeldung: 20.10.1993
(73) Patentinhaber: UNI-CHARM COMPANY LIMITED, Kawanoe-Shi, Ehime 799-01 (JP)
(72) Erfinder: SUZUKI, Migaku, Kanagawa 247 (JP); YAMAMOTO, Masamitsu, Ehime 799-01 (JP); MURAKAMI, Masaki, Kawanoe-shi Ehime 799-01 (JP)
(74) Vertreter: Sperling, Rüdiger, Dipl.-Ing.
(86) Internationale Anmeldenummer: JP9101520
(87) Internationale Veröffentlichungsnummer: WO9308780

(56) Entgegenhaltungen:
- JP-A-62 057 975
- JP-A-62 161 364
- US-A- 2 902 037
- US-A- 4 601 868

## Beschreibung

Die vorliegende Erfindung betrifft eine flexible luftdurchlässige Kunststoffolie mit einem gewebtem Stoff ähnlichen Erscheinungsbild.

Es ist bekannt, eine flexible thermoplastische Folie einschließlich einer Kunststoffolie mit einer Vielzahl feiner Poren zu versehen und dadurch die Folie luftdurchlässig zu machen. Die auf diese Weise geformte poröse Kunststoffolie hat gewöhnlich den für Kunststoffolien kennzeichnenden schmierigen Glanz und klebrigen Griff, die bei den Benutzern bei einigen Anwendungsgebieten der Folie auf Abneigung stoßen. Um einen derartigen Glanz abzumildern und den Griff der Folie zu verbessern, wurden bereits verschiedene Techniken vorgeschlagen, beispielsweise eine Technik, durch die die Folienoberfläche geprägt wird, um den Glanz wegzunehmen und gleichzeitig die Oberfläche mit Unregelmäßigkeiten und Poren zu versehen, und eine Technik, durch die ein dritter Inhaltsstoff mit abstumpfender sowie porenbildender Wirkung vorab dem Rohmaterial für die Folie beigemischt wird, so daß die gewünschte Glanzabstumpfung und Porenformungwirkung bei einem Schritt der Folienherstellung erzielt werden können.

Diese bekannten Techniken waren jedoch bisher nicht in der Lage, eine zufriedenstellende Verbesserung der Kunststoffolie insbesondere bei derartigen Kunststoffolien zu erreichen, die als Decklage von am Körper zu tragenden Wegwerfhygieneartikeln, wie zum Beispiel Damenbinden und Wegwerfwindeln verwendet werden, da dieser Glanz und Griff der Decklage derartiger tragbarer Hygieneartikel, die zur direkten Berührung mit der Haut des Trägers bestimmt sind, entschieden abgelehnt werden. Das US Patent Nr. 4,342,314 zeigt eine Technik auf, um eine flexible Kunststoffolie mit einem faserähnlichen Erscheinungsbild und einer Kapillarstruktur zu versehen. Diese Technik verfolgt ebenso die Absicht, den gewöhnlich die Kunststoffolie kennzeichnenden klebrigen Griff abzumildern. Die Oberfläche der durch diese Technik erhaltenen Folie wird jedoch als glatt betrachtet und aufgrund ihrer Glattheit als einen eher abstoßenden Glanz aufweisend.

Demgemäß ist es eine Hauptaufgabe der vorliegenden Erfindung, den Glanz und unerwünschten Griff, der herkömmlicherweise Kunststoffolien kennzeichnet, abzumildern, indem die Kunststoffolie mit luftdurchlässigen Leerräumen versehen wird, die durch einander überkreuzende Rippen begrenzt sind, so daß diese Rippen und Leerräume zusammenwirken können, um ein gewebtem Stoff ähnliches Erscheinungsbild zu schaffen.

Die vorstehend dargelegte Aufgabe wird gemäß vorliegender Erfindung durch eine flexible, luftdurchlässige Kunststoffolie mit einem gewebtem Stoff ähnlichen Erscheinungsbild erzielt, umfassend:
eine Vielzahl von in einer ersten Richtung verlaufenden Rippen, die jeweils einander gegenüberliegende, nach unten gekrümmte Seitenränder aufweisen, und eine Vielzahl von in einer zweiten Richtung verlaufenden Rippen, die jeweils einander gegenüberliegende, nach unten gekrümmte Seitenränder aufweisen, so daß diese Rippen erster Richtung und Rippen zweiter Richtung einander überkreuzen, um das gewebtem Stoff ähnliche Erscheinungsbild zu bieten;
einen luftdurchlässigen Leerraum, den jedes Paar benachbarter Rippen erster Richtung und jedes Paar benachbarter Rippen zweiter Richtung, die die Rippen erster Richtung überkreuzen, zusammen begrenzt;
wobei das Rippenpaar erster Richtung an ersten und zweiten Kreuzungspunkten, die benachbart und einander diagonal gegenüberliegend angeordnet sind, nach oben vorgewölbt ist, so daß in erster Richtung ausgerichtete Wölbungen ausgebildet werden, und einerseits die beiden unteren Ränder jeder Vorwölbung mit den Oberseiten des Rippenpaares zweiter Richtung verbunden sind, und das Rippenpaar zweiter Richtung an dritten und vierten benachbarten und diagonal einander gegenüberliegend angeordneten Kreuzungspunkten nach oben vorgewölbt ist, so daß in zweiter Richtung ausgerichtete Vorwölbungen gebildet werden, und andererseits die beiden unteren Ränder jeder der Vorwölbungen mit den Oberseiten des Rippenpaares erster Richtung verbunden sind.

Gemäß einem Aspekt der Erfindung haben die Rippen erster Richtung und die Rippen zweiter Richtung jeweils vorzugsweise einen hohlen bogenförmigen Querschnitt.

Gemäß einem weiteren Aspekt der Erfindung haben die Rippen zweiter Richtung jeweils vorzugsweise eine geringere Breite als die jeweiligen Rippen erster Richtung, wenigstens entlang einem in Längsrichtung ausgerichteten Mittelbereich der Rippen zweiter Richtung.

Gemäß einem weiteren Aspekt der Erfindung ist das Paar benachbarter Rippen erster Richtung vorzugsweise mit die einander gegenüberliegenden unteren Ränder dieser Rippen verbindenen Brücken versehen.

Durch die Erfindung geschaffene vorteilhafte Auswirkungen sind aus folgender Beschreibung ohne weiteres ersichtlich.

Bei der erfindungsgemäßen Kunststoffolie mit vorstehend beschriebenem Aufbau überkreuzen die Rippen erster Richtung die Rippen zweiter Richtung und an jedem Kreuzungspunkt wölbt sich eine der Rippen nach oben vor, so daß jede Rippe einen wellenförmigen Verlauf zwischen jedem Paar diese Rippe überkreuzender Rippen aufweist und ein Erscheinungsbild schafft, das der Struktur von Gewebe mit Leinwandbindung entspricht. Das Vorsehen der Brücken zwischen jedem Paar benachbarter Rippen schafft ein Erscheinungsbild, das der Struktur von Gewebe mit Köperbindung entspricht.

Der hohle bogenförmige Querschnitt jeder Rippe verleiht der Rippe eine Pufferwirkung und verleiht dadurch der gesamten Kunststoffolie einen angenehmeren bauschigen Griff.

Die Rippen erster Richtung und die Rippen zweiter Richtung sind jeweils in verschiedenen Abständen angeordnet, um die Rippenanordnungen zu komplizieren und dadurch ein komplizierteres Erscheinungsbild der Kunststoffolie ähnlich dem von gewebtem Stoff zu schaffen.

Eine komplizierte Variation der Kunststoffolienoberfläche durch Vorwölbungen und Wellenstrukturen jeder Rippe fördert die diffuse Reflexion einfallenden Lichtes und mildert dadurch in wirksamer Weise den herkömmlicherweise für Kunststoffolien allgemein kennzeichnenden abstoßenden Glanz.

Die Vorwölbungen und die Wellenstruktur auf der Oberfläche der Kunststoffolie verringern in wirksamer Weise die Oberfläche der zur Berührung mit der Haut des Trägers bestimmten Folie und mildern dadurch den klebrigen Griff, der ebenfalls herkömmlicherweise für Kunststoffolien kennzeichnend ist.

Nachfolgend wird die Erfindung anhand von Figuren näher beschrieben.
Fig. 1 bis 6 sind vergrößerte perspektivische Draufsicht- und Schnittdarstellungen, die teilweise eine Ausführungsform einer flexiblen, luftdurchlässigen Kunststoffolie zeigen, die gemäß der Lehre der Erfindung aufgebaut ist;
Fig. 7 und 8 sind vergrößerte ausgebrochene Draufsichten, die alternative Ausführungsformen einer derartigen Kunststoffolie zeigen;
Fig. 9 bis 11 sind vergrößerte Teildraufsicht- und Teilschnittdarstellungen, die eine weitere Ausführungsform einer derartigen Kunststoffolie zeigen;
Fig. 12 ist eine vergrößerte Teildraufsicht einer weiteren Ausführungsform einer derartigen Kunststoffolie; und
Fig. 13 ist eine perspektivische, teilweise ausgebrochene Darstellung der erfindungsgemäßen Kunststoffolie in einer beispielhaften Verwendung in einer Damenbinde.

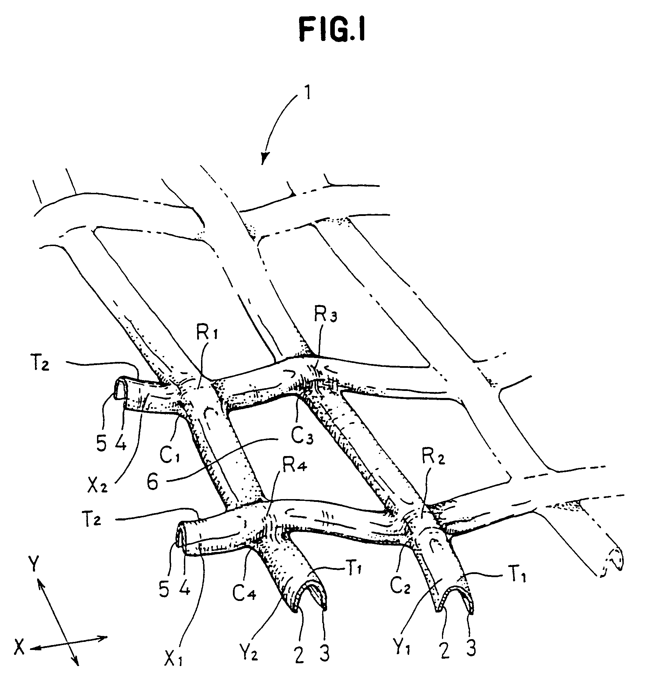

In Fig. 1 bis 6 ist eine Ausführungsform der flexiblen, luftdurchlässigen Kunststoffolie 1, die gemäß der Lehre der Erfindung aufgebaut ist, teilweise in Drauf- bzw. Schnittdarstellungen gezeigt.

In Fig. 1 stellen durchgezogene Linien die kleinste Einheit der zahllose Rippen umfassenden Folie 1 dar und diese Einheit ist in Längs- und Querrichtung wiederholt, um eine vollständige Folie 1 zu bilden. Diese Einheit der Folie 1 umfaßt ein in einer ersten Richtung Y verlaufendes Rippenpaar Y₁, Y₂ und ein in einer zweiten Richtung X verlaufendes Rippenpaar X₁, X₂, wobei diese Rippen eine im wesentlichen gleichförmige Breite aufweisen. Die jeweiligen Rippen sind in Querrichtung zur Unterseite der Folie 1 hin umgebogen, so daß die Rippen Y₁, Y₂ jeweils zwei in Längsrichtung parallel zueinander verlaufende untere Ränder 2, 3 und die Rippen X₁, X₂ in ähnlicher Weise jeweils zwei in Längsrichtung parallel zueinander verlaufende untere Ränder 4, 5 aufweisen. Die beiden benachbarten, in erster Richtung verlaufenden Rippen Y₁, Y₂ überkreuzen die beiden in zweiter Richtung verlaufenden, benachbarten Rippen X₁, X₂ an Kreuzungspunkten C₁ bis C₄. An den einander benachbarten und diagonal gegenüberliegend angeordneten ersten und zweiten Kreuzungspunkten C₁, C₂ wölben sich die Rippen erster Richtung Y₁, Y₂ nach Oben und bilden jeweils eine Vorwölbung R₁ bzw. R₂. In ähnlicher Weise wölben sich die Rippen zweiter Richtung X₁, X₂ nach oben und bilden an den einander benachbarten und gegenüberliegend angeordneten dritten und vierten Kreuzungspunkten C₃, C₄ Vorwölbungen R₃ bzw. R₄. An den Vorwölbungen R₁, R₂ sind die jeweiligen beiden unteren Ränder 2, 3 der Rippen erster Richtung Y₁, Y₂ mit den jeweiligen Oberseiten T₂ der Rippen zweiter Richtung X₁, X₂ verbunden, die in der zweiten Richtung verlaufen. In ähnlicher Weise sind an den Vorwölbungen R₃, R₄ die jeweiligen beiden unteren Ränder 4, 5 der Rippen zweiter Richtung X₁, X₂ mit den jeweiligen Oberseiten T₁ der Rippen erster Richtung Y₁, Y₂, die in erster Richtung verlaufen, verbunden. Die Rippen Y₁, Y₂ und die benachbarten Rippen X₁, X₂ treffen im wesentlichen rechtwinklig aufeinander und diese vier Rippen begrenzen zusammen einen luftdurchlässigen Leerraum 6, der eine Durchgangsöffnung bildet. Die in dieser Weise aufgebaute Folie 1 hat ein Erscheinungsbild, das dem von Gewebe mit Leinwandbindung entspricht.

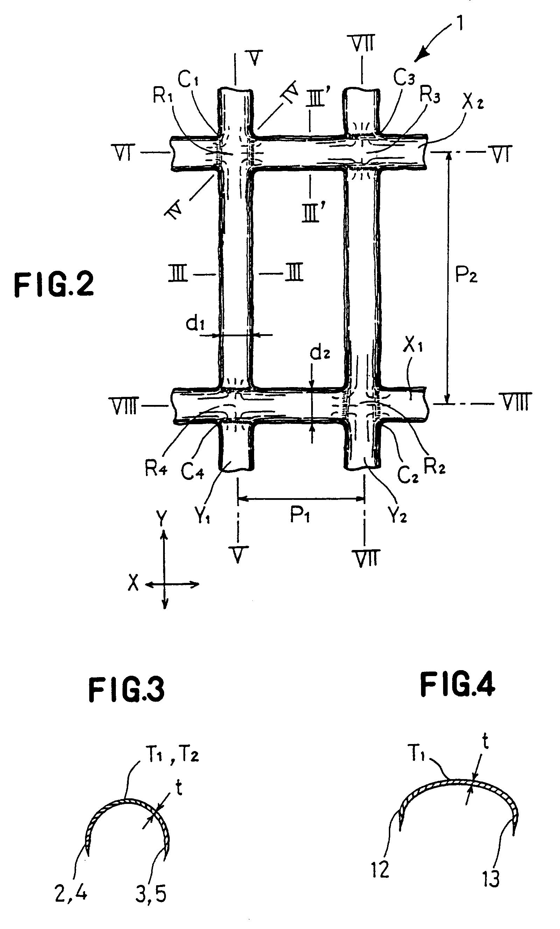

Fig. 2 ist eine Draufsicht auf die in Fig. 1 gezeigte Folie 1. Wie in dieser Figur dargestellt, haben die Rippen erster Richtung Y₁, Y₂ einen Abstand P₁ und die Rippen zweiter Richtung X₁, X₂ einen Abstand P₂, wobei P₁ < P₂. Die Folie 1 kann jedoch auch so aufgebaut sein, daß eine Beziehung von P₁ ≧ P₂ entsteht. Darüberhinaus können die Rippen einander schräg überkreuzen anstatt sich im rechten Winkel zu kreuzen.

Die Rippen Y₁, Y₂ haben eine Breite d₁ und die Rippen X₁, X₂ eine Breite d₂. Während diese spezifische Ausführungsform so dargestellt ist, daß die Breite d₁ im wesentlichen gleich der Breite d₂ ist, kann einer der Werte d₁ oder d₂ größer sein als der andere und jede Rippe kann entlang ihrem in Längsrichtung ausgerichteten Mittelbereich örtlich verjüngt sein.

Fig. 3 ist eine allgemeine schematische Schnittdarstellung der Rippe Y₁ entlang einer Linie III-III und der Rippe X₂ entlang einer Linie III'-III' in Fig. 2. Wie dargestellt hat jede Rippe im wesentlichen einen halbkreisförmigen Querschnitt, der die Oberseite T₂ oder T₁ umfaßt, eine im wesentlichen gleichförmige Stärke t und zwei keilförmig sich verjüngende untere Ränder 2, 3 (an der Rippe Y₁) oder 4, 5 (an der Rippe X₂). In Fig. 4 ist die Rippe erster Richtung Y₁ am Kreuzungspunkt C₁ in einer Schnittdarstellung entlang einer in einem Winkel von 45° zur Rippe Y₁ verlaufenden Linie IV-IV in Fig. 2 dargestellt. Die Rippe Y₁ zeigt einen im wesentlichen halbelliptischen Querschnitt einschließlich der Oberseite T₁, eine Stärke t und zwei keilförmige untere Ränder 12, 13, entlang welchen die Oberseite T₂ und die angrenzenden Bereiche mit den unteren Rändern 2, 3 der Rippe Y₁ verbunden sind. Die entlang in einem Winkel von 45° bezüglich der ersten Richtung an den anderen Kreuzungspunkten C₂ bis C₄ verlaufenden Linien abgenommenen Querschnitte sind im wesentlichen mit dem in Fig. 4 dargestellten identisch, mit der Ausnahme, daß die jeweiligen Rippen die Vorwölbungen bilden.

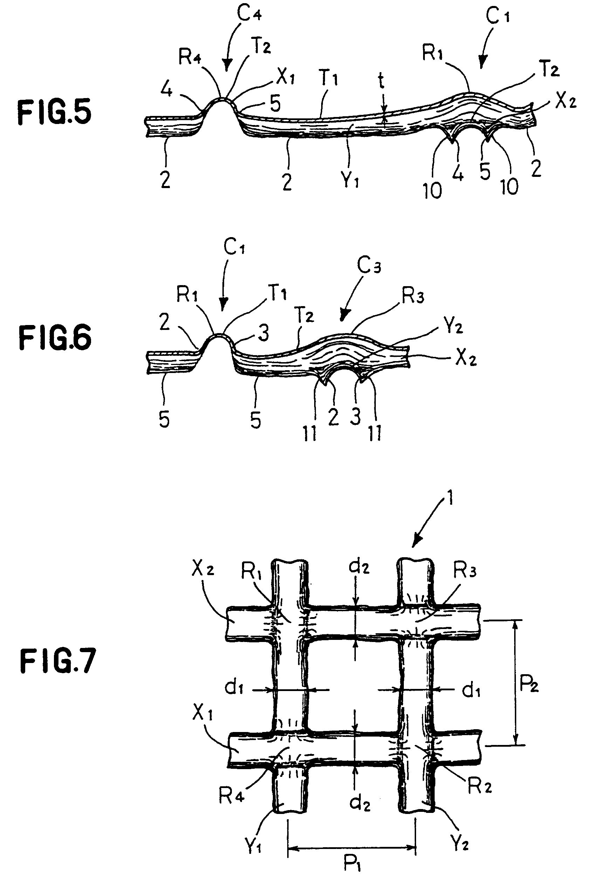

Fig. 5 ist eine Schnittdarstellung entlang einer Linie V-V in Fig. 2 und schließt Schnitte der Vorwölbungen R₁, R₄ an den Kreuzungspunkten C₁ bzw. C₄ jeweils ein. Am Kreuzungspunkt C₁ wölbt sich die Rippe Y₁ auf und bildet so die Vorwölbung R₁ und der untere Rand 2 derselben ist mit der Oberseite T₂ der Rippe X₂ verbunden. An den einander gegenüberliegenden Seiten der Vorwölbung R₁ ist in Fig. 5 ersichtlich, daß die unteren Ränder 4, 5 der Rippe X₂ durch Randlinien 10 mit dem unteren Rand 2 der Rippe Y₁ verbunden sind. Am Kreuzungspunkt C₄ wölbt sich die Rippe X₁ zur Vorwölbung R₄ auf und die unteren Ränder 4, 5 derselben sind mit der Oberseite T₁ der Rippe Y₁ verbunden. Es ist ebenfalls offensichtlich, daß die Rippe Y₁ zwischen den Rippen X₁, X₂ in Wellenlinie verläuft.

Fig. 6 ist eine Schnittdarstellung entlang einer Linie VI-VI in Fig. 2 und umfaßt Schnittdarstellungen der Vorwölbungen R₁, R₃ an dem Kreuzungspunkt C₁ bzw. C₃. Die Weise, in der die Rippen Y₁ und X₂ sich nach oben wölben und miteinander am Kreuzungspunkt C₁ verbunden sind, wurde bereits unter Bezug auf Fig. 5 beschrieben. Am Kreuzungspunkt C₃ wölbt sich die Rippe X₂ zur Aufwölbung R₃ nach oben und der untere Rand 5 derselben ist mit der Oberseite T₁ der Rippe Y₂ verbunden. An den einander gegenüberliegenden Seiten der Wölbung R₃ sind die unteren Ränder 2, 3 der Rippe Y₂ durch Randlinien 11 mit den unteren Rändern 5 der Rippe X₂ verbunden.

Entlang Linien VII-VII und VIII-VIII in Fig. 2 jeweils genommene Schnittdarstellungen entsprechen im wesentlichen einer seitenverkehrten Darstellung von Fig. 5 bzw. 6 und sind der Einfachheit halber nicht dargestellt.

Um ein einem Gewebe entsprechendes Erscheinungsbild der Folie 1 zu erreichen, sollte die Breite der Rippen d₁, d₂ 0,01 bis 3 mm betragen, vorzugsweise 0,05 bis 2 mm, und die Anzahl der Leerräume 6 sollte 5 bis 90 pro 25,4 mm, vorzugsweise 10 bis 60 pro 25,4 mm der jeweiligen Rippen betragen. Die Folie 1 kann aus 5 bis 50 g/m², vorzugsweise 20 bis 30 g/m² Polyethylen, Polypropylen, Polyester oder anderen thermoplastischen Folien hergestellt sein. Eine Verbesserung des Glanzes wie auch des Griffs und eine weitere Hervorhebung des faserähnlichen Erscheinungsbildes werden in effektiver Weise durch verschiedene Maßnahmen erzielt, wie etwa eine rauhe Oberflächenbearbeitung der Folie 1 zur Verbesserung der diffusen Reflexion von einfallendem Licht, die Ausbildung der Konturen jedes Leerraumes 6 als unregelmäßige Krümmungen, unregelmäßige Variation der Rippenbreite in Längsrichtung derselben, geeignete Veränderung des Rippenabstandes und eine geeignete Färbung der Folie 1.

Fig. 7 zeigt eine alternative Ausführungsform der Erfindung in Draufsicht. Wie in der Figur dargestellt, hat jede Rippe Y₁, Y₂ eine Breite d₁, die im wesentlichen gleich einer Breite d₂ jeder Rippe X₁, X₂ ist, und die Rippen Y₁, Y₂ haben einen Abstand P₁, der im wesentlichen gleich einem Abstand P₂ der Rippen X₁, X₂ ist.

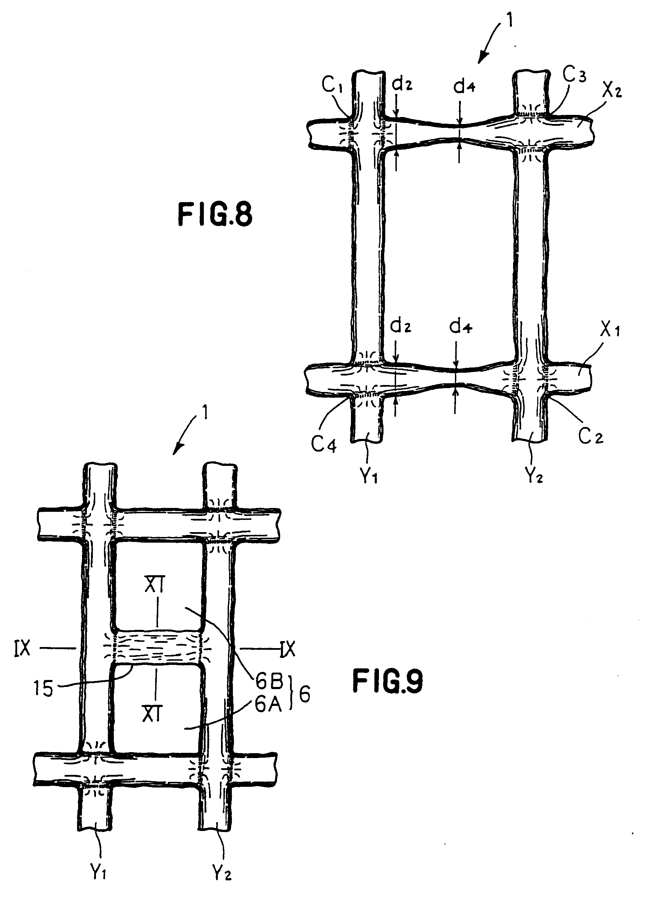

Fig. 8 zeigt eine weitere alternative Ausführungsform der Erfindung ebenfalls in Draufsicht. In dieser bestimmten Kunststoffolie 1 verändern sich die Breiten d₂ der jeweiligen Rippen X₁, X₂ kontinuierlich zwischen den Kreuzungspunkten C₁ und C₃ bzw. zwischen den Kreuzungspunkten C₂ und C₄. Genauer weisen die Rippen X₁, X₂ die am stärksten verringerte Breite d₄ jeweils an den Mittelpunkten auf.

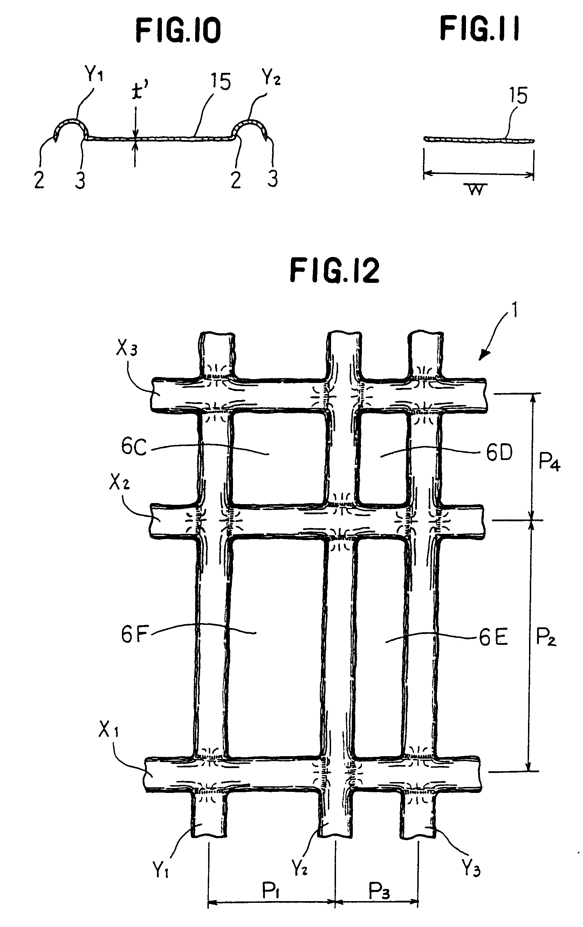

In Fig. 9 bis 11 ist noch eine weitere Ausführungsform in Draufsicht bzw. Schnittdarstellung gezeigt. Wie Fig. 9 zeigt, enthält diese Kunststoffolie 1 eine Brücke 15, die die Rippen Y₁, Y₂ miteinander verbindet. Fig. 10 und Fig. 11 sind Schnittdarstellungen entlang Linien IX-IX und XI-XI in Fig. 9. Die Brücke 15 erstreckt sich zwischen dem unteren Rand 3 der Rippe Y₁ und dem gegenüberliegend angeordneten unteren Rand 2 der Rippe Y₂ und hat eine Breite W und eine Stärke t'. Die Breite W kann zwischen den entgegengesetzten Enden der Brücke 15 unregelmäßig variieren und die Stärke t' ist vorzugsweise im wesentlichen gleich der oder kleiner als die Stärke t der Rippe, so daß die gewünschte Flexibilität der Folie 1 aufrecht erhalten werden kann. Es ist nicht erforderlich, alle Leerräume 6 mit derartigen Brücken 15 zu versehen. In dieser bestimmten Ausführungsform ergibt die Brücke 15 zusammen mit den benachbarten Rippen ein einem Gewebe mit Köperbindung entsprechendes Erscheinungsbild.

In Fig. 12 ist eine weitere Ausführungsform in Draufsicht dargestellt. Die Rippen Y₁, Y₂, Y₃ haben in der ersten Richtung Abstände P₁, P₃, während die Rippen X₁, X₂, X₃ Abstände P₂, P₄ haben, wobei diese Abstände sich voneinander unterscheiden. Mit anderen Worten sind die Rippen erster Richtung (Y₁, Y₂, Y₃ ...) in abwechselnd wiederholten Abständen P₁, P₃ angeordnet und die Rippen zweiter Richtung (X₁, X₂, X₃ ...) in den abwechselnd wiederholten Abständen P₂, P₄ angeordnet, um so die vollständige Folie 1 zu bilden.

Die Kunststoffolie 1 kann durch verschiedene Verfahren hergestellt werden, wobei eines der relativ einfachen Verfahren auf dem Prinzip der Thermoformung basiert, das die Schritte des Auflegens einer Kunststoffolie in thermisch erweichtem Zustand auf die Oberseite eines Drahtnetzes mit einer durch Draht gebildeten Struktur ähnlich Leinwandbindung, Köperbindung, Satinbindung oder ähnlichem, Beaufschlagen der Kunststoffolie mit einer von der Unterseite des Drahtnetzes angelegten Vakuumsaugwirkung und Versehen der Kunststoffolie mit Poren an den den Maschen des Drahtnetzes entsprechenden Stellen durch Zerreißen der Kunststoffolie an diesen Stellen unter dem Effekt der Vakuumsaugwirkung umfaßt. Zur kontinuierlichen Herstellung der Kunststoffolie 1 kann eine drehbare Trommel an ihrer äußeren Umfangsfläche mit dem Drahtnetz versehen sein, wobei die drehbare Trommel zur Beaufschlagung mit Vakuumsaugwirkung an vorgegebenen Stellen an der äußeren Umfangsfläche eingerichtet sein kann und eine Rolle von Folienmaterial kann, während die Folie erweicht wird, kontinuierlich auf die drehbare Trommel abgewickelt werden. Die von der Trommel abgewickelte Kunststoffolie 1 kann anschließend in Querrichtung gedehnt werden, um die durch die Rippen begrenzten Leerräume der Poren zu vergrößern, die Rippenbreiten zu vergrößern oder die unregelmäßig sich ändernden Rippenbreiten zu erhalten. Beispielsweise kann die in Fig. 8 dargestellte Folie 1 durch Dehnen der in Fig. 1 dargestellten Folie 1 erhalten werden. Die Folie kann vorab durch Erwärmen erweicht werden, wenn dies für den Dehnungsschritt erforderlich ist. Ein derartiges Verfahren führt oftmals dazu, daß die Folie 1 eine örtlich ungleichmäßige Konfiguration aufweist, und es versteht sich, daß eine derartige Folie 1 ebenfalls vom Umfang der Erfindung gedeckt ist. Zusätzlich kann das Drahtnetz wenigstens teilweise aus Litzendraht gebildet sein und die Folie kann gegen ein derartiges Drahtnetz unter der Vakuumsaugwirkung gepreßt werden, um feine Unregelmäßigkeiten auf den Rippenoberflächen zu erzielen, die mit den Litzendrähten während der Thermoformung in engen Kontakt gekommen sind.

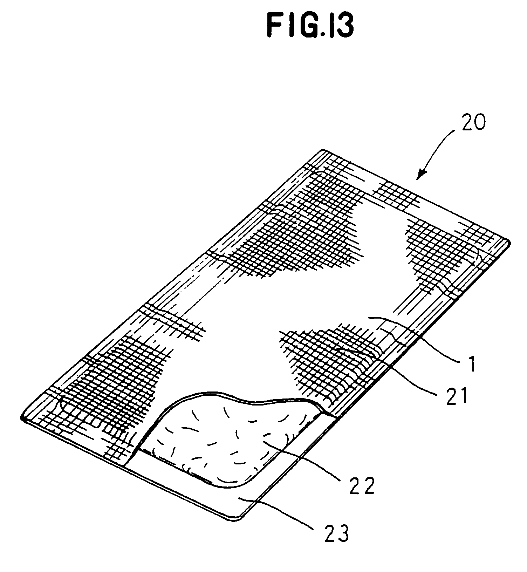

Fig. 13 zeigt in teilweise ausgebrochener perspektivischer Darstellung eine Damenbinde 20, bei der die Kunststoffolie 1 als Decklage 21 verwendet wird. Die Binde 20 umfaßt einen absorbierenden Kern 22, der zum Absorbieren und Zurückhalten von Menstruationsflüssigkeit eingerichtet ist, eine luft- und flüssigkeitsdurchlässige Decklage 21 und eine flüssigkeitsundurchlässige Außenlage 23. Die Decklage 21 umfaßt die in Fig. 1 gezeigte Kunststoffolie 1, der Absorptionskern 22 umfaßt eine Mischung aus Faserpulpe und hoch wasserabsorbierendem Polymerpulver und die Außenlage 23 umfaßt Polyethylenfolie. Die Decklage 21 und die Außenlage 23 sind entlang den vier Rändern der Binde 20 miteinander verbunden, um die Menstruationsflüssigkeit zurückzuhalten. Die unteren Ränder 2 bis 5 der die Kunststoffolie 1 bildenden Rippen sind nach innen gerichtet und stehen mit dem Absorptionskern 22 in Berührung.

Die gemäß der Lehre der vorliegenden Erfindung aufgebaute Kunststoffolie ist unter den folgenden Gesichtspunkten industriell verwendbar:
1. Die Folie weist auf ihrer Oberfläche feine Wölbungen und Wellenstrukturen auf, an denen die diffuse Reflexion von einfallenden Lichtstrahlen auftritt, um den für Kunststoffolien herkömmlicherweise allgemein kennzeichnenden, in gewisser Weise abstoßenden Glanz abzumildern, und die Fläche der Folie zu verringern, die zur Berührung mit der Haut des Trägers bestimmt ist, um so den in gewisser Weise klebrigen Griff zu eliminieren, der ebenfalls herkömmlicherweise für Kunststoffolien allgemein kennzeichnend ist.
2. Die Folie zeigt in Abhängigkeit vom Muster der Rippenanordnung ein Erscheinungsbild der Oberfläche, das einer Struktur von gewebtem Stoff, wie etwa Leinwandbindung, Köperbindung oder ähnlichem entspricht.
3. Jede Rippe ist hohl mit halbkreisförmigem Querschnitt ausgeführt, was zum dämpfenden und bauschigen Griff beiträgt.

Wenn die erfindungsgemäße Folie als flüssigkeitsdurchlässige Decklage für einen Hygiene-Wegwerfartikel, wie zum Beispiel Damenbinden verwendet wird, bietet die Folie einen angenehmen Griff und auf diese Folie ausgetragene Körperflüssigkeiten werden rasch entlang den Rippen mit halbkreisförmigen Querschnitten nach unten durch die Leerräume in einen Absorptionskern fließen, um so das Stocken der Körperflüssigkeiten in der Decklage zu vermeiden.

## Patentansprüche

1. Flexible, luftdurchlässige Kunststoffolie mit einem gewebtem Stoff ähnlichen Erscheinungsbild, umfassend:
eine Vielzahl von in einer ersten Richtung (Y) verlaufenden Rippen (Y₁,Y₂), die jeweils einander gegenüberliegende, nach unten gekrümmte Seitenränder (2, 3) aufweisen, und eine Vielzahl von in einer zweiten Richtung (X) verlaufenden Rippen (X₁, X₂), die jeweils einander gegenüberliegende, nach unten gekrümmte Seitenränder (4, 5) aufweisen, so daß diese Rippen erster Richtung (Y₁, Y₂) und Rippen zweiter Richtung (X₁, X₂) einander überkreuzen, um das gewebtem Stoff ähnliche Erscheinungsbild zu bieten;
einen luftdurchlässigen Leerraum (6), den jedes Paar benachbarter Rippen erster Richtung (Y₁, Y₂) und jedes Paar benachbarter Rippen zweiter Richtung (X₁, X₂), die die Rippen erster Richtung (Y₁, Y₂) überkreuzen, zusammen begrenzt;
wobei das Rippenpaar erster Richtung (Y₁, Y₂) an ersten und zweiten Kreuzungspunkten (C₁, C₂), die benachbart und einander diagonal gegenüberliegend angeordnet sind, nach oben vorgewölbt ist, so daß in erster Richtung (Y) ausgerichtete Wölbungen (R₁, R₂) ausgebildet werden, und einerseits die beiden unteren Ränder jeder Vorwölbung (R₁, R₂) mit den Oberseiten (T₂) des Rippenpaares zweiter Richtung (X₁, X₂) verbunden sind, und das Rippenpaar zweiter Richtung (X₁, X₂) an dritten und vierten benachbarten und diagonal einander gegenüberliegend angeordneten Kreuzungspunkten (C₃, C₄) nach oben vorgewölbt ist, so daß in zweiter Richtung (X) ausgerichtete Vorwölbungen (R₃, R₄) gebildet werden, und andererseits die beiden unteren Ränder jeder der Vorwölbungen (R₃, R₄) mit den Oberseiten (T₁) des Rippenpaares erster Richtung (X₁, X₂) verbunden sind.

2. Kunststoffolie nach Anspruch 1,
wobei jede der Rippen erster Richtung (Y₁, Y₂) und der Rippen zweiter Richtung (X₁, X₂) einen hohlen, bogenförmigen Querschnitt aufweist.

3. Kunststoffolie nach Anspruch 1 oder 2,
wobei jede Rippe zweiter Richtung (X₁, X₂) wenigstens entlang einem in Längsrichtung mittleren Abschnitt eine geringere Breite (d₄) als die Breite (d₁) jeder der Rippen erster Richtung (Y₁, Y₂) aufweist.

4. Kunststoffolie nach Anspruch 1, 2 oder 3,
wobei jedes benachbarte Rippenpaar erster Richtung (Y₁, Y₂) mit Brücken (15) versehen ist, die einander gegenüberliegende untere Ränder (2, 3) dieser Rippen verbinden.

5. Kunststoffolie nach Anspruch 1, 2, 3 oder 4,
wobei die Rippen erster Richtung (Y₁, Y₂) und die Rippen zweiter Richtung (X₁, X₂) eine im wesentlichen gleichförmige Breite (d₁, d₂) aufweisen.

6. Kunststoffolie nach Anspruch 1, 2, 3, 4 oder 5,
wobei die Rippen erster Richtung (Y₁, Y₂) in einem Abstand (P₁) angeordnet sind, der größer ist als der Abstand (P₂), in dem die Rippen zweiter Richtung (X₁, X₂) angeordnet sind.

7. Kunststoffolie nach Anspruch 1, 2, 3, 4, 5 oder 6, wobei eine Oberfläche der Folie (1) ein einem Gewebe mit Leinwandbindung entsprechendes Erscheinungsbild bietet.

8. Kunststoffolie nach Anspruch 1, 2, 3, 4, 5 oder 6, wobei eine Oberfläche der Folie (1) ein einem Gewebe mit Köperbindung entsprechendes Erscheinungsbild bietet.

## Claims

1. Flexible, air-permeable plastic foil with an appearance similar to woven material, comprising:
a plurality of ribs (Y₁, Y₂) running in a first direction (Y) which each have downwardly curved opposing side edges (2, 3), and a plurality of ribs (X₁, X₂) running in a second direction (X) which each have downwardly curved opposing side edges (4, 5), so that these ribs in the first direction (Y₁, Y₂) and ribs in the second direction (X₁, X₂) cross over one another to provide the appearance similar to woven material;
an air-permeable space (6) bordered by each pair of adjacent ribs in the first direction (Y₁, Y₂) and each pair of adjacent ribs in the second direction (X₁, X₂) crossing over the ribs in the first direction (Y₁, Y₂) together;
wherein the rib pair in the first direction (Y₁, Y₂) is arched forwards and upwards at the first and second intersection points (C₁, C₂) arranged adjacent and diagonally opposite one another, so that arched portions (R₁, R₂) directed in the first direction (Y) are formed, and on the one hand the two lower edges of each forward arched portion (R₁, R₂) are joined to the upper sides (T₂) of the rib pair in the second direction (X₁, X₂), and the rib pair in the second direction (X₁, X₂) is arched forwards and upwards at the third and fourth intersection points (C₃, C₄) arranged adjacent and diagonally opposite one another, so that forward arched portions (R₃, R₄) directed in the second direction (X) are formed, and on the other hand the two lower edges of each of the forward arched portions (R₃, R₄) are joined to the upper sides (T₁) of the rib pair in the first direction (Y₁, Y₂).

2. Plastic foil according to Claim 1, wherein each of the ribs in the first direction (Y₁, Y₂) and of the ribs in the second direction (X₁, X₂) has a hollow arc-shaped cross-section.

3. Plastic foil according to Claim 1 or 2, wherein, at least along a central section in the longitudinal direction, the width (d₄) of each rib in the second direction (X₁, X₂) is smaller than the width (d₁) of each of the ribs in the first direction (Y₁, Y₂).

4. Plastic foil according to Claim 1, 2 or 3, wherein each adjacent rib pair in the first direction (Y₁, Y₂) is provided with bridges (15) which join opposing lower edges (2, 3) of these ribs.

5. Plastic foil according to Claim 1, 2, 3 or 4, wherein the ribs in the first direction (Y₁, Y₂) and the ribs in second direction (X₁, X₂) have a substantially uniform width (d₁, d₂).

6. Plastic foil according to Claim 1, 2, 3, 4 or 5, wherein the ribs in the first direction (Y₁, Y₂) are spaced at a distance (P₁) which is greater than the distance (P₂) at which the ribs in second direction (X₁, X₂) are spaced.

7. Plastic foil according to Claim 1, 2, 3, 4, 5 or 6, wherein one surface of the foil (1) has an appearance corresponding to a fabric with linen weave.

8. Plastic foil according to Claim 1, 2, 3, 4, 5 or 6, wherein one surface of the foil (1) has an appearance corresponding to a fabric with twill weave.

## Revendications

1. Film de matière plastique souple et perméable à l'air, offrant un aspect semblable à celui d'une étoffe tissée, comprenant :
- une pluralité de côtes (Y1, Y2) s'étendant dans une première direction (Y) et qui présentent des bords latéraux (2, 3) en opposition mutuelle et recourbés vers le bas, et une pluralité de côtes (X1, X2) s'étendant dans une seconde direction (X) et qui présentent des bords latéraux (4, 5) en opposition mutuelle et recourbés vers le bas, de sorte que ces côtes de premier sens (Y1, Y2) et ces côtes de second sens (X1, X2) s'entrecroisent, pour offrir un aspect semblable à celui d'une étoffe tissée ;
- un vide (6) perméable à l'air délimitant entre elles deux côtes de premier sens (Y1, Y2) voisines, et deux côtes de second sens (X1, X2) voisines s'entrecroisant avec lesdites côtes de premier sens (Y1, Y2) ; film dans lequel les deux côtes de premier sens (Y1, Y2) sont précintrées vers le haut à la hauteur d'un premier et d'un second point de croisement (C1, C2) voisins et diagonalement opposés, de sorte que des cintrages (R1, R2) orientés dans le premier sens sont produits, et que, d'une part, les deux bords inférieurs de chaque précintrage (R1, R2) sont reliés aux faces supérieures (T2) des deux côtes de second sens (X1, X2), et dans lequel les deux côtes de second sens (X1, X2) sont précintrées vers le haut à la hauteur d'un troisième et d'un quatrième point de croisement (C3, C4) voisins et diagonalement opposés, de sorte que des précintrages (R3, R4) orientés dans le second sens (X) sont produits, et que, d'autre part, les deux bords inférieurs de chacun des précintrages (R3, R4) sont reliés aux faces supérieures (T1) de la paire de côtes de premier sens (Y1, Y2).

2. Film de matière plastique selon la revendication 1, dans lequel chacune des côtes de premier sens (Y1, Y2) et des côtes de second sens (X1, X2), présente une section transversale creuse en forme d'arc.

3. Film de matière plastique selon l'une des revendications 1 ou 2, dans lequel chaque côte de second sens (X1, X2) présente une largeur (d4) inférieure à la largeur (d1) de chacune des côtes de premier sens (Y1, Y2), au moins sur une portion médiane dans le sens de la longueur.

4. Film de matière plastique selon l'une quelconque des revendications 1, 2 ou 3, dans lequel chaque paire de côtes de premier sens (Y1, Y2) voisine présente des ponts (15) reliant deux bords inférieurs (2, 3) mutuellement opposés de ces côtes.

5. Film de matière plastique selon l'une quelconque des revendications 1, 2, 3 ou 4, dans lequel les côtes de premier sens (Y1, Y2) et les côtes de second sens (X1, X2) présentent une largeur (d1, d2) sensiblement uniforme.

6. Film de matière plastique selon l'une quelconque des revendications 1, 2, 3, 4 ou 5, dans lequel les côtes de premier sens (Y1, Y2) sont espacées d'une distance (P1) qui est supérieure à la distance (P2) dont sont espacées les côtes de second sens (X1, X2).

7. Film de matière plastique selon l'une quelconque des revendications 1, 2, 3, 4, 5 ou 6, dans lequel une surface du film (1) offre un aspect correspondant à un tissu ayant une armure de toile.

8. Film de matière plastique selon l'une quelconque des revendications 1, 2, 3, 4, 5 ou 6, dans lequel une surface du film (1) offre un aspect correspondant à un tissu ayant une armure de sergé.
